(19) Europäisches Patentamt
European Patent Office
Office européen des brevets

(11) Numéro de publication: **0 105 821 B1**

# (12) FASCICULE DE BREVET EUROPEEN

(45) Date de publication du fascicule du brevet: **26.02.86**

(21) Numéro de dépôt: **83450024.1**

(22) Date de dépôt: **30.09.83**

(51) Int. Cl.⁴: **C 07 D 263/28**, C 07 D 413/06, C 07 D 413/14, A 61 K 31/42, A 61 K 31/435

## (54) Nouvelles N-(aminométhyl-5-oxazolin-2-yl-2)N'-phénylurées.

(30) Priorité: **05.10.82 FR 8216783**
**04.08.83 FR 8313013**

(43) Date de publication de la demande: **18.04.84 Bulletin 84/16**

(45) Mention de la délivrance du brevet: **26.02.86 Bulletin 86/9**

(84) Etats contractants désignés: **BE CH DE GB IT LI LU NL**

(56) Documents cités:
**DE - B - 1 302 662**
**FR - M - 3 206**
**GB - A - 1 196 544**

(73) Titulaire: **SOCIETE CORTIAL S.A., 7 rue de l'Armorique, F-75015 Paris (FR)**

(72) Inventeur: **Creuzet, Marie-Hélène, Résidence Jardin de Gambetta T3, F-33000 Bordeaux (FR)**
Inventeur: **Feniou, Claude, 5 rue du Général Guillomat, F-33600 Pessac (FR)**
Inventeur: **Jarry, Christian, 11 rue des Mésanges, F-33370 Artigues près Bordeaux (FR)**
Inventeur: **Prat, Gisèle, Hameau de Noailles Villa 18, F-33400 Talence (FR)**
Inventeur: **Pontagnier, Henri, 21 rue Edouard Vaillant, F-33600 Pessac (FR)**

(74) Mandataire: **Tajan, Marie-Thérèse, LABORATOIRES SARGET Avenue du Président JF Kennedy, F-33701 Mérignac (FR)**

EP 0 105 821 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des N-(aminométhyl 5 oxazolin-2 yl-2)N'-phénylurées, leur méthode de préparation ainsi que leur application thérapeutique.

Les produits de la présente invention ont pour formule générale

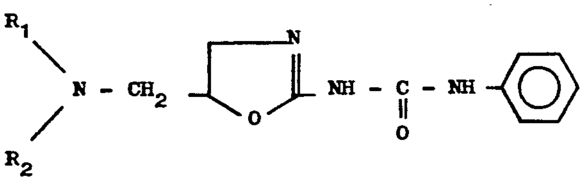

avec $R_1$, $R_2$ identiques ou différents = alkyl (choisi parmi $CH_3$, $C_2H_5$, $C_3H_7$, $C_4H_9$) ou benzyl ou phényl; $R_1$ et $R_2$ peuvent former avec l'atome d'azote auquel ils sont rattachés un hétérocycle choisi parmi pipéridine, pyrrolidine, morpholine, tétrahydroisoquinoléïne, ou bien pipéridine ou pipérazine substituée par R avec

R = alkyl inférieur de Cl à C4, allyl, benzyl, pyridyl, phényl substitué ou non par un ou plusieurs substituants choisi parmi halogène (par exemple chloro, fluoro, bromo), trifluorométhyl, méthyl, méthoxy, hydroxy.

On connait déjà des amino-2 oxazolines-2. Ainsi l'amino-2 phényl-5 oxazoline-2

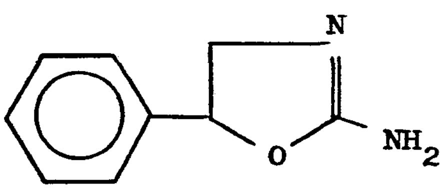

a été brevetée par la Société Mac Neil Incorporated en France sous le et son activité anorexigéne. L'amino-2 (dichloro-3,4 phénoxyméthyl)-5 oxazoline-2 a été testée par A.H. Abdallah et coll. pour son activité cardiovasculaire et anorexigàne (Toxicol. appl. Pharmacol., 1973, 26, 513-22; 1973, 25, 344-53) et a été brevetée par la Société Dow Chemical aux Etats Unis sous le n° 3637726 le 9 avril 1970 pour son activité antimicrobienne.

On connait également un dérivé de substitution de l'urée de formule

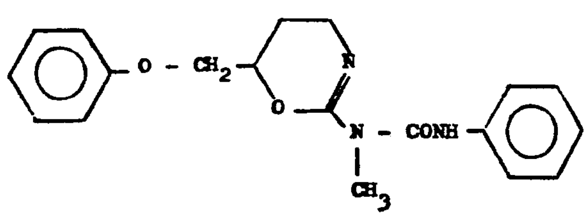

décrit par la Société Delalande dans le brevet français n° 7311985.

Les produits de la présente invention se distinguent des dérivés déjà connus par la présence en position 5 du cycle oxazoline d'un substituant leur application en thérapeutique notamment dans le traitement des convulsions, des troubles du rythme, de la maladie ulcéreuse et des états inflammatoires ou oedémateux.

Les produits de la présente invention sont préparés de iaçon générale par réaction entre une amino-2 aminométhyl-5 oxazoline-2 et l'isocyanate de phényle dans un solvant tel que le benzène à la température d'ébullition du solvant.

L'invention va être décrite plus précisément dans les exemples suivants sans toutefois que ceux ci ne limitent sa portée.

### Exemple 1

N-(diéthylaminométhyl-5 oxazolin-2 yl-2) N'-phénylurée. Produit de formule I avec $R_1 = R_2 = C_2H_5$.

Dans un réacteur muni d'un réfrigérant, d'une ampoule à brome et d'une agitation, on introduit 17,1 g d'amino-2 diéthylaminométhyl-5 oxazoline-2 à ébullition et 11,9 g d'isocyanate de phényle sont ajoutés goutte à goutte. Le chauffage est prolongé deux heures aprés la fin de l'addition d'isocyanate de phényle.

Lors du refroidissement il se forme un précipité qui est essoré et recristallisé dans $CCl_4$. Rendement 92 %. Point de fusion 148°C. RMN dans DMSOD6 (sont donnés les déplacements chimiques exprimés en ppm par rapport au TMS pris comme étalon interne) 0,9 ppm, 6 protons, triplet ($CH_3$); 2,3 -2,8 ppm, 6 protons, massif complexe, ($CH_2N$); 3,2-4,0 ppm, 2 protons, massif complexe, (H en 4 du cycle oxazoline); 4,4-5,0 ppm, 1 proton, massif complexe (H en 5 du cycle oxazoline); 6,7-7,8 ppm, 5 protons, massif complexe, (protons aromati9ues); 8,7 et 9,3 ppm, 2 protons, dômes, (NHCONH).

### Exemple 2

N-phényl N'-(pipéridinométhyl-5 oxazolin-2 yl-2) urée; Formule I avec $R_1$ et $R_2$ ensemble = $-CH_2-CH_2-CH_2-CH_2-CH_2-$.

Ce produit est préparé selon la technique décrite dans l'exemple 1. Point de fusion 170°C.

RMN dans $CDCl_3$ 1,2-1,8 ppm, 6 protons, massif complexe ($CH_2C$); 2,2-2,8 ppm, 6 protons, massif complexe ($CH_2N$); 3,3-4,0 ppm, 2 protons, massif complexe (H en 4 du cycle oxazoline);

4,5-5 1 ppm, 1 proton massif complexe (H en 5 du cycle oxazoline);

6 8-7 7 ppm 5 protons massif complexe (protons aromatiques); 8,2 et 8,8 ppm, 2 protons, dômes (NHCONH).

**Exemple 3**

N-phényl N'-(tétrahydro-1,2,3,4 isoquinoléinométhyl-5 oxazolin-2 yl-2) urée; formule I avec $R_1$ et $R_2$ ensemble =

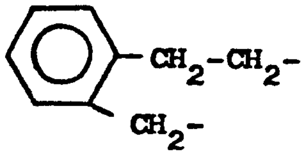

Ce produit est préparé selon la technique décrite dans l'exemple 1. Point de fusion 179°C.

RMN dans DMSOD6

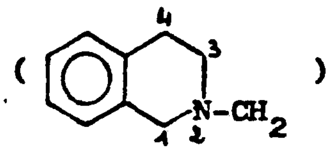

2,6-3,1 ppm, 6 protons, massif complexe (H en 3,4 du cycle isoquinoléïne + $CH_2$ en alpha); 3,2-4,0 ppm, 4 protons, massif complexe (H en 1 du cycle isoquinoléïne + H en 4 du cycle oxazoline); 4,6-5,1 ppm, 1 proton, massif complexe (H en 5 du cycle oxazoline); 6,6-7,7 ppm, 9 protons, massif complexe (H aromatiques); 8,7-9,3 ppm, 2 protons, dômes (HHCONH).

**Exemple 4**

N-((méthyl-4 pipérazinylméthyl)-5 oxazolin-2y1-2) N'-phénylurée. Produit de formule 1 avec $R_1NR_2$ =

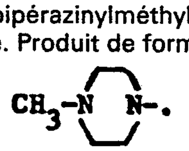

Ce produit est préparé selon la technique décrite dans l'exemple 1.

Point de fusion 150° C (banc Kofler).

Spectre de RMN dans le DMSOD6, (déplacements chimiques exprimés en ppm par rapport au TMS pris comme étalon interne). 2,0-2,8 ppm, 13 protons, massif complexe, méthylpipérazinylméthyl dont $CH_3$ à 2,1 ppm; 3,2-4,0 ppm, 2 protons, massif complexe, $CH_2$ du cycle oxazoline; 4,5-5,1 ppm, 1 proton, multiplet, CHO; 6,7-7,8 ppm, 5 protons, massif complexe, protons aromatiques; 8,7 et 9,3 ppm, 2 protons, dômes, 2 NH, échangeables avec $D_2O$.

**Exemeple 5**

N-((benzyl-4 pipéridinylméthyl)-5 oxazolin-2y1-2) N'-phénylurée. Produit de formule 1 avec

$R_1NR_2 = C_6H_5CH_2$ - 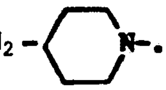

Ce produit

est préparé selon l'exemple 1.

Point de fusion 181° C (banc Kofler).

Spectre de RMN dans le DMSOD6: 0,9-4,0 ppm, 15 protons, massif complexe, $CH_2$ et CH; 4,5-5,0 ppm, 1 proton, multiplet, CHO; 6,7-7,8 ppm, 10 protons, massif complexe, protons aromatiques; 8,7 et 9,3 ppm, 2 protons, dômes, 2NH, échangeables avec $D_20$.

**Exemple 6**

N-((benzyl-4 pipérazinylméthyl)-5 oxazolin-2y1-2) N'-phénylurée. Produit de formule I avec $R_1NR_2$

= $C_6H_5CH_2$ - 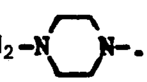

Ce produit est préparé selon l'exemple 1.

Point de fusion 184° C (banc Kofler).

Spectre de RMN dans le DMSOD6: 2,2-2,8 ppm, 10 protons, massif complexe, $CH_2$ pipérazinylméthyl; 3,2-4,0 ppm, 4 protons, massif complexe, $CH_2$ du cycle oxazoline + $CH_2$ benzylique; 4,5-5,1 ppm, 1 proton, multiplet, CHO; 6,7-7,8 ppm, 10 protons, massif complexe, protons aromatiques; 8,8 et 9,3 ppm, 2 protons, dômes, 2NH, échangeables avec $D_20$.

Les propriétés toxicopharmacologiques des produits de la présente invention sont exposées ci-après.

La toxicité a été déterminée chez la souris pour diverses voies d'administration. Ainsi les produits des exemples 1, 2 et 3 n'entrainent aucune mortalité quand ils sont administrés par voie orale à 300 mg/kg ou par voie intrapéritonéale à 200 mg/kg.

Administrés par voie intrapéritonéale à la dose de 100 mg/kg les produits des exemples 1 et 2 empêchent l'apparition d'arythmies chez des souris soumises à des anesthésies au chloroforme.

Administré par voie intrapéritonéale à la dose de 80 mg/kg le produit de l'exemple 3 entraine une inhibition des convulsions induites par une injection de 0,5 mg/kg de bicuculline 30 mn après. Le produit de l'exemple 3 n'agit pas sur la Gaba transaminase car il n'inhibe plus les convulsions induites par une seconde administration de bicuculline 5 h après. Son activité est donc du type gabergique direct.

A la concentration de 100 microg/ml le produit de l'exemple 1 inhibe à plus de 50 % l'effet

chronotrope induit par 5 microg/ml d'histamine sur l'oreillette droite de cobaye battant spontanément.

Administré par voie orale à la dose de 200 mg/kg le produit de l'exemple 1 entraine une inhibition de 51 % de l'oedéme de la patte du rat induit par la carragénine.

Compte tenu de leurs activités pharmacologiques, les produits de la presente invention peuvent être employés en thérapeutique humaine et vétérinaire. Associés aux excipients habituels ils pourront être utilisés par exemple pour traiter les troubles du rythme cardiaque, les épilepsies généralisées ou localisées, les convulsions fébriles de l'enfant, la maladie ulcéreuse, les états inflammatoires et oedémateux.

Ils seront administrés par exemple par voie orale sous forme de dragées, comprimés, sirop, ampoules, par voie rectale sous forme de suppositoires, par voie intramusculaire ou intraveineuse ou par voie ou bien pipéridine ou pipérazine substiuée par R avec R = alkyl inférieur de C1 à C4, allyl, benzyl, pyridyl, phényl substitué ou non par un ou plui;eurs substituants choisi parmi halogène (par exemple chloro, fluoro, bromo), trifluorométhyl, méthyl, méthoxy, hydroxy.

3 - Médicament caractérisé en ce que le principe actif est constitué par au moins un produit selon la revendication 1.

4 - Médicament selon la revendication 3 utile pour le traitement des troubles du rythme cardiaque.

5 - Médicament selon la revendication 3 utile pour le traitement des épilepsies et convulsions.

6 - Composition pharmaceutique ou vétérinaire caractérisée en ce qu'elle contient à titre de principe actif au moins un produit selon la revendication 1 en association avec un véhicule pharmaceutique ou un excipient approprié.

**Revendications**

1 - Produits de formule générale

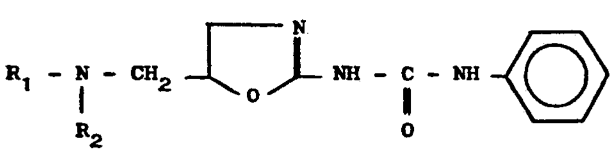

avec $R_1$, $R_2$ ident;ques ou d;fférents = alkyl (chois; parmi $CH_3$, $C_2H_5$, $C_3H_7$, $C_4H_9$) ou benzyl ou phényl; R1 et R2 peuvent former avec l'atome auquel ils sont rattachés un hétérocycle choisi parmi p;pér;d;ne, pyrrolidine, morpholine, tétrahydroisoquinoléïne, ou bien pipéridine ou pipérazine substituée par R avec R = alkyl inférieur de C1 à C4, allyl, benzyl, pyridyl, phényl substitué ou non par un ou plusieurs subst;tuants choisi parmi halogène (par exemple chloro, fluoro, bromo), trifluorométhyl, méthyl, méthoxy, hydroxy.

2 - Méthode de préparation des produits selon la revendication 1 caractérisée en ce que l'on fait réagir dans un solvant choisi parmi le benzène, à la température d'ébullition du solvant, l'isocyanate de phenyle avec un dérivé de formule générale

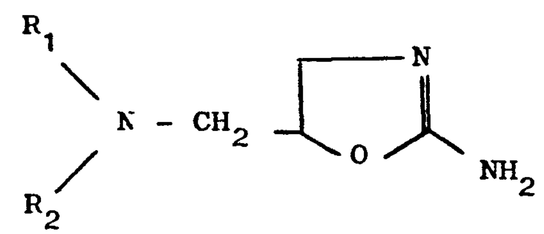

dans laquelle $R_1$, $R_2$ identiques ou différents = alkyl (choisi parmi $CR_3$, $C_2H$, $C_3H_7$ $C_4H_9$) ou benzyl phényl; R1 et R2 peuvent former avec l'atome d'azote auxqiels ils sont rattachés un hétérocycle choisi parmi pipéridine, pyrrolidine, morpholine, tetrahydroisoquinoleïne, topique sous forme de pommade ou de gel; les doses administrées varieront selon l'indication et le sujet de 1 à 100 mg/j en 2 à 6 prises pour la voie orale, de 1 à 100 mg/j en 1 ou 2 prises pour la voie rectale, de 0,5 à 50 mg par injection pour les voies parentérales.

**Claims**

1. Products with the general formula

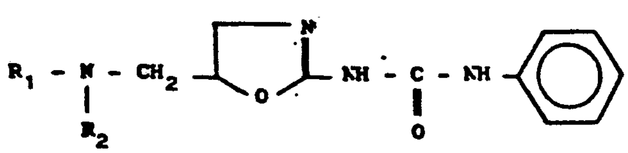

with $R_1$, $R_2$, identical or different = alkyl (selected among $CH_3$, $C_2H_5$, $C_3H_7$, $C_4H_9$) or benzyl or phenyl; $R_1$ and $R_2$ may form together with that atom they are joined to, a heterocycle selec ted among piperidine. pyrrolidine, morpholine, tetrahydroisoquinolein, or else piperidine or piperazine substituted by R, with H = lower alkyl from C1 to C4, allyl, benzyl, pyridyl, phenyl, substituted or not by one or a plurality of substituents selected among halogen (for example chloro-, fluoro-, bromo-), triiluoromethyl, methyl, methoxy, hydroxy.

2. Method for preparing the products as defined in claim 1, in which one reacts in a solvent selected among the benzene. at the solvent boiling temperature, phenyl isocyanate with a derivative having as general formula

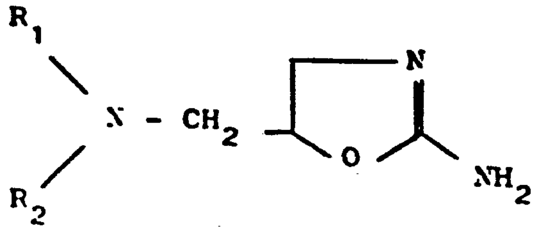

in which $R_1$, $R_2$, identical or different, = alkyl (selected among and $R_2$ may form together with that nitrogen atom they are joined to, a heterocycle selected among piperidine, pyrrolidine, morpholine, tetraisoquinoleine. or else piperidine or piperazine substituted by R, with R = lower alkyl from C1 to C4, allyl, benzyl, pyridyl, phenyl substituted or not by one or a plurality of substituents selected among halogen (for example chloro-, fluoro-, bromo-), trifluoromethyl, methyl, methoxy, hydroxy.

3. Medicament, in which the active constituent is comprised of at least one product as defined in claim 1.

4. Medicament as defined in claim 3, useful for treating disorders in the heart rhythm.

5. Medicament as defined in claim 3, useful for treating epilepsies and spasms.

6. Pharmaceutic or veterinary compound, which contains as active constituent, at least one product as defined in claim 1 in association with a suitable pharmaceutic vehicle or excipient.

**Patentansprüche**

1. Verbindungen der allgemeinen Formel

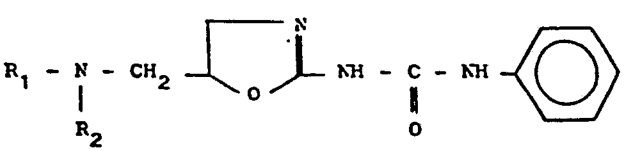

in der $R_1$ und $R_2$, die gleich oder verschieden sein können, Alkyl (ausgewählt aus $CH_3$, $C_2H_5$, $C_3H_7$, $C_4H_9$), Benzyl oder Phenyl bedeuten oder mit dem N-Atom, an das sie gebunden sind, einen Heterocyclus, ausgewählt aus der von Piperidin, Pyrrolidin, Morpholin, Tetrahydroisochinolin, oder Piperidin oder Piperazin, jeweils substituiert mit einer Gruppe R, wobei R = niedriges $C_1$-$C_4$-Alkyl, Allyl, Benzyl, Pyridyl oder gegebenenfalls mit einem oder mehreren Substituenten aus der Gruppe Halogen (z.B. Chlor, Fluor, Brom) Trifluormethyl, Methyl, Methoxy oder Hydroxy substituiertes Phenyl, bedeutet, bilden.

2. Verfahren zur Herstellung der Verbindungen nach Anspruch 1, dadurch gekennzeichnet, daß man in einem Lösemittel wie Benzol beim Siedepunkt des Lösemittels Phenylisocyanat mit einem Derivat der allgemeinen Formel

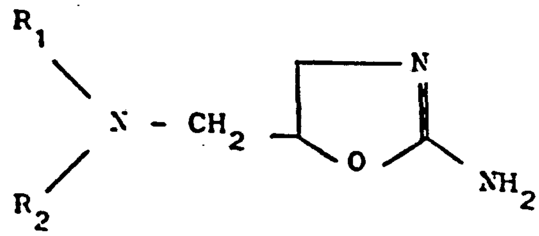

in der $R_1$ und $R_2$, die gleich oder verschieden sein können, Alkyl (ausgewählt aus $CH_3$, $C_2H_5$, $C_3H_7$, $C_4H_9$), Benzyl oder Phenyl bedeuten oder mit dem N-Atom, an das sie gebunden sind, einen Heterocyclus, ausgewählt aus der von Piperidin, Pyrrolidin, Morpholin, Tetrahydroisochinolin, oder PiPeridin, oder Piperazin, jeweils substituiert mit einer Grupp R, wobei R = niedriges $C_1$-$C_4$Alkyl, Allyl, Benzyl, Pyridyl, oder gegebenenfalls mit einem oder mehreren Substituenten aus der Gruppe Halogen (z.B. Chlor, Fluor, Brom), Trifluormethyl, Methyl, Methoxy oder Hydroxy substituiertes Phenyl, bedeutet, bilden, umsetzt.

3. Medikament, dadurch gekennzeichnet, daß sein Wirkstof f aus mindestens einer Verbindung nach Anspruch 1 besteht.

4. Medikament nach Anspruch 3 für die Behandlung von Herzrhythmusstörungen.

5. Medikament nach Anspruch 3 für die Behandlung von Epilepsien und Konvulsionen.

6. Pharmazeutische oder veterinärmedizinische Zusammensetzung, dadurch gekennzeichnet, daß sie als Wirkstoff mindestens eine Verbindung nach Anspruch 1 zusammen mit einem pharmazeutischen Träger oder einem geeigneten Exzipienten enthält.